# EUROPEAN PATENT APPLICATION

(11) **EP 1 997 479 A1**
(43) Date of publication of application: **03.12.2008**
(21) Application number: 07010802.2
(22) Date of filing: 31.05.2007
(51) Int. Cl.: A61K 9/14, A61K 9/16, A61K 9/20, A61K 31/4184, A61K 9/28, A61K 31/54

(54) **Stabilized amorphous candesartan cilexetil compositions for oral administration**

(71) Applicant: Helm AG, 20097 Hamburg (DE)
(72) Inventor: Gindullis, Frank, Dr., 25365 Klein Offenseth-Sparrieshoop (DE); Löffler, Uwe, Dr., 25436 Tornesch (DE); Stritzke, Katja, Dr., 20259 Hamburg (DE)
(74) Representative: Becker Kurig Straus

(57) **Abstract**

The present invention relates to stabilized amorphous candesartan cilexetil compositions for oral administration which have enhanced dissolution and reduced degradation characteristics, and to an efficient and economical process for the preparation of these compositions. Another aspect of the invention relates to stabilized amorphous candesartan cilexetil compositions, obtainable by said process, as pharmaceutical formulations prepared while employing said stabilized amorphous candesartan cilexetil compositions. The stabilized amorphous candesartan cilexetil compositions according to the present invention have greatly improved chemical stability. Preferred drug formulations according to the present invention are tablets, capsules, pellets, and granules prepared with usual, pharmaceutically acceptable excipients in ways known per se. Particularly preferred according to the invention are tablets which rapidly release the active ingredient that are prepared by direct compression of the stabilized amorphous candesartan cilexetil compositions obtained according to the present invention.

## Description

### BACKGROUND OF THE INVENTION

### 1. Field of the Invention

The present invention relates to stabilized amorphous candesartan cilexetil compositions for oral administration which have enhanced dissolution and reduced degradation characteristics, and to an efficient and economical process for the preparation of these compositions. Another aspect of the invention relates to stabilized amorphous candesartan cilexetil compositions, obtainable by said process, as pharmaceutical formulations prepared while employing said stabilized amorphous candesartan cilexetil compositions. The stabilized amorphous candesartan cilexetil compositions according to the present invention have greatly improved chemical stability. Preferred drug formulations according to the present invention are tablets, capsules, pellets, and granules prepared with usual, pharmaceutically acceptable excipients in ways known per se. Particularly preferred according to the invention are tablets which rapidly release the active ingredient that are prepared by direct compression of the stabilized amorphous candesartan cilexetil compositions obtained according to the present invention.

### 2. Description of Related Art

The active ingredient having the chemical name of (±)-1-(Cyclohexyloxycarbonyloxy)ethyl 2-ethoxy-1-[[2'-(1H-tetrazol-5-yl)biphenyl-4-yl]methyl]-1 H-benzimidazole-7-carboxylate, also known by the INN as candesartan cilexetil, can be represented by the following structural formula:

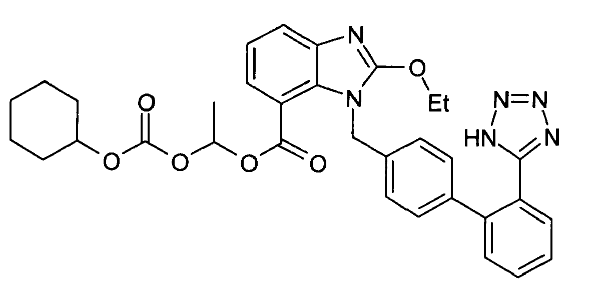

Candesartan cilexetil belongs to the class of non-peptide angiotensin-II receptor antagonists having a very high selectivity for the AT1 receptors. Candesartan cilexetil is a pro-drug and it is hydrolyzed to candesartan during absorption from the gastrointestinal tract. It belongs to the class of benzimidazole-7-carboxylic acid derivatives. These agents exhibit a strong and more effective hypotensive action and are less likely to cause coughing as a side effect as compared to other classes of ACE inhibitors. With a common daily dose of 2 to 32 mg, candesartan cilexetil is used as a single-substance preparation (mono-preparation) or in combination with the diuretic hydrochlorothiazide for the treatment of cardiovascular diseases. As an AT1 receptor antagonist, candesartan cilexetil more particularly inhibits the rise of the blood pressure caused by angiotensin II, suppresses angiotensin-II-induced aldosterone secretion, and lowers angiotensin-II-induced liquid uptake (*see, for instance,* Arzneimittelwirkung: Lehrbuch der Pharmakologie und Toxikologie, E. Mutschler, G. Geisslinger, H.K. Kroemer, M Schäfer-Korting, editors, 8th edition, Wissenschaftliche Verlagsgesellschaft mbH Stuttgart, 2001*).*

Candesartan cilexetil, its pharmaceutically acceptable salts and processes for its preparation are described in EP 0 459 136 B1, working examples 2 to 8. The preparation of candesartan cilexetil in the C-type crystalline form and in an amorphous form is also described in EP 0 459 136 B1. Candesartan cilexetil of the type C crystalline form (form C) is obtained by crystallization from lower alcohols (methanol, ethanol), a mixture of a lower alcohol and water, and a mixture of a lower alkyl ketone (like acetone) and water. Amorphous candesartan cilexetil can be obtained by column chromatography purification of crude candesartan cilexetil and evaporation of the solvent. The amorphous powder has been reported to be unstable to heat and impractical in production (experimental example 1). Two crystalline forms of candesartan cilexetil, form I and form II, are described in Chem. Pharm. Bull. 47 (2), 182-186 (1999).

In WO 2004/085426 a candesartan cilexetil 1,4-dioxane solvate, prepared by dissolving candesartan cilexetil in dioxane and crystallization at a temperature of 5-15°C, is disclosed. This application further comprises candesartan cilexetil form III crystallized from toluene, and candesartan cilexetil form IV which is crystallized from a mixture of MTBE and methanol. The disclosed polymorphic forms of this application have been characterized by their x-ray diffraction patterns.

WO 2005/077941 discloses a large variety of polymorphic forms and solvates of candesartan cilexetil and processes for their preparation. Forms II, III, IV, V, VI, VII, VIII, IX, X, XI, XIII, XIV, XIV-1, XV, XVI, XVII, XVIII, XIX, XX, XXI, XXII, XXIII, and amorphous candesartan cilexetil are part of the application.

WO 2005/123721 describes an amorphous form of candesartan cilexetil having less than about 600 ppm residual dichloromethane solvent, which is prepared by spray drying.

In the solid state, pure candesartan cilexetil is stable against moderate heat, moisture and light. Candesartan cilexetil has low solubility in water because it is hydrophobic in nature. However, when candesartan is formulated into tablets with other ingredients it has been observed that the content of the active ingredient decreases over time. Especially O-desethyl candesartan cilexetil is formed as a degradation product.

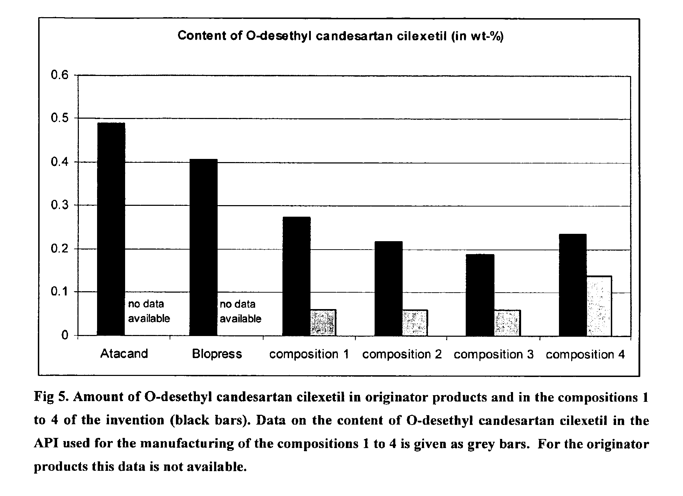

In several analyses of the originator candesartan cilexetil tablets, considerable amounts of O-desethyl candesartan cilexetil have been be detected (e.g., for Atacand^{®} or Blopress^{®} 8 mg tablets, the amount of O-desethyl candesartan cilexetil is > 0.4wt %) (see Fig. 5 below).

Since the low bioavailability of hydrophobic drugs with extremely low water solubility can be a serious problem, different approaches have been taken to achieve the desirable high levels of drug solubility and dissolution rate.

EP 0 546 358 B1 discloses that the reduction in the content of active candesartan cilexetil in pharmaceutical compositions with the lapse of time can be reduced by incorporating oily substances having a low melting point. The oily substances are incorporated into the active component to form a stable composition in which decomposition with time caused by compression is suppressed.

In EP 1 441 705 A1 a process is disclosed for the preparation of stable solid or semisolid formulations starting from liquid active principles which undergo prompt or modified release from the system are disclosed. The active principles are stabilized by the use of amphiphilic and/or lipophilic matrices characterized by melting at temperatures ranging from 30°C to 90°C and by being solids at room temperatures to at least to 25°C.

WO 2004/071494 deals with pharmaceutical compositions containing biologically active compounds with low water solubility complexed with lipophilic polymers to increase the solubility in aqueous media. The therapeutic agent and lipophilic polymer are homogeneously dispersed in a water-miscible organic solvent and precipitated by the addition of water or removal of the solvent.

In WO 2005/070398 stable pharmaceutical compositions for oral administration including candesartan cilexetil and one or more solvents and co-solvents are provided. The co-solvent is selected from propylene glycol, polyethylene alcohol, ethanol, glycerol, propylene glycol esters, or polyethylene glycol esters. Optionally the composition may comprise a fatty acid ester like glycerol stearate, glycerol palmitate, glyceryl caprate, glyceryl caprylate, glycerol oleate, glycerol linoleate, or glyceryl lauropalmitoleate.

WO 2005/079751 discloses pharmaceutical compositions that include candesartan cilexetil together with one or more fatty substances which are present at concentrations of between 0.5% and 10% w/w. The fatty substances may be lipids and phospholipids.

In WO 2005/084648 a pharmaceutical composition including candesartan cilexetil and one or more water-soluble polymers is described. The pharmaceutical composition contains a water-soluble polymer selected from polyvinyl alcohol, maltodextrin, xanthan gum, polyvinylpyrrolidone, hydroxypropyl cellulose, hydroxypropyl methyl cellulose, hydroxyethyl cellulose, sodium carboxymethylcellulose, calcium carboxymethylcellulose, methyl cellulose, tragacanth gum, agar, gellan gum, karaya gum, alginic acids, pectins, or pregelatinized starch.

Finally, WO 2005/123720 provides candesartan cilexetil having a particle size with a D_{0.9} value of about 25 µm or less. According to the application the pharmaceutical solid composition comprising candesartan cilexetil of reduced particle size exhibits a significantly improved pharmacokinetic profile and good bioavailability. The small particle sized candesartan cilexetil is prepared by specific crystallization, grinding, milling, jet milling, media milling, or pulverization. The inventors state that pharmaceutical solid formulations containing candesartan cilexetil with D_{0.9} values from about 70 µm to about 40 µm exhibit a pharmacokinetic profile which is unpredictable.

Currently known processes for the preparation of stable pharmaceutical formulations containing amorphous candesartan cilexetil, which itself is said to be unstable, require a considerable technical effort and are time and cost intensive, and satisfactory stabilization may not be reached at all. In addition, none of the above mentioned formulations contains amorphous candesartan cilexetil, and therefore these preparations are not completely satisfactory since all of them require additional effort in order to improve the dissolution behavior.

Thus, it is the object of the present invention to provide stable and readily soluble candesartan cilexetil formulations in a short, efficient and simple process. By this process, it should be possible to prepare the solid pharmaceutical formulation starting from any polymorphic form or particle size of candesartan cilexetil or its hydrates and solvates. The solid pharmaceutical formulations of stabilized amorphous candesartan cilexetil compositions should exhibit greatly improved dissolution and chemical stability properties.

### BRIEF SUMMARY OF THE INVENTION

The object of the present invention is to develop a simple and economical process for the preparation of stabilized amorphous candesartan cilexetil compositions that can be used immediately for manufacturing pharmaceutical formulations.

### BRIEF DESCRIPTION OF THE DRAWINGS

FIG. 1 shows the powder X-ray diffraction pattern of candesartan cilexetil crystalline form C;
FIG. 2 shows the powder X-ray diffraction pattern of a stabilized candesartan cilexetil composition with Eudragit^{®} E PO according to the present invention, composition 1;
FIG. 3 shows the powder X-ray diffraction pattern of a stabilized candesartan cilexetil composition with Eudragit^{®} E PO according to the present invention, composition 2;
FIG. 4 shows powder X-ray diffraction patterns of stabilized candesartan cilexetil composition in Eudragit^{®} E PO, lactose, microcrystalline cellulose and polyvinylpyrrolidone according to the present invention, composition 4;
FIG. 5 presents a bar chart showing the amount of O-desethyl candesartan in Atacand^{®}, and Blopress^{®} compared to compositions 1 to 4 of candesartan cilexetil according to the present invention, and to the pure candesartan cilexetil product obtained according to EP 0 459 136 B1;
FIG. 6 shows a comparative dissolution profile of the pure crystalline candesartan cilexetil and the compositions 2 and 5 of the present invention obtained in phosphate buffer pH6.5 + 0.35% Tween 20.

### DETAILED DESCRIPTION OF THE INVENTION

The present invention relates to a process for the preparation of stabilized amorphous candesartan cilexetil compositions which have an enhanced dissolution rate and an improved impurity profile regarding degradation products. In the stabilized amorphous candesartan cilexetil compositions which can be obtained by said process the amorphous candesartan cilexetil can be present both in the anhydrous form and/or in the form of solvates or hydrates.

The stable compositions produced according to the invention contain amorphous candesartan cilexetil and methacrylate based polymers.

The invention further relates to pharmaceutical formulations containing these novel stabilized amorphous candesartan cilexetil compositions. Where applicable, the pharmaceutical formulations may contain further excipients and optionally further pharmaceutical suitable excipients and may be converted to the desired specific drug delivery formulations. Tablets produced by direct compressing which rapidly release the active ingredient are a particularly preferred formulation.

The pharmaceutical formulations according to the invention which contain candesartan cilexetil as the AT1 receptor antagonist can be employed to treat diseases including for instance the following examples:
(a) Hypertension, cardiac insufficiency, renal failure, particularly chronic renal failure, restenosis after percutaneous transluminal angioplasty, and restenosis after coronary arterial bypass surgery,
(b) arteriosclerosis, insulin resistance and syndrome X, diabetes mellitus type 2, obesity, nephropathy, renal failure, for instance chronic renal failure, hypothyreosis, the condition after cardiac infarction, coronary cardiopathy, age-related hypertension, familial dyslipidemic hypertension, and all the precited diseases in connection with or without hypertension,
(c) endothelial dysfunction in connection with or without hypertension,
(d) hyperlipidemia, hyperlipoproteinemia, arteriosclerosis and hypercholesterinemia,
(e) glaucoma.

The pharmaceutical compositions of the present invention contain at least one stabilizing agent, i.e. an agent which stabilizes candesartan cilexetil in its amorphous form and reduces its chemical degradation. In the following, these compositions of amorphous candesartan cilexetil with at least one stabilizing agent will be referred to as "stabilized amorphous candesartan cilexetil compositions".

Optionally, at least one pharmaceutically acceptable excipient may be additionally present in the composition and in its formulations. Excipients which are insoluble or poorly soluble in organic solvents are used. The excipients may be selected from celluloses and cellulose derivatives, lactose, starches and starch derivatives, cyclodextrins, polydextroses or mixtures of said substances. Microcrystalline cellulose, lactose, and mannitol are preferred according to the invention. For an improvement of the flow properties, additives containing silica or titania can be used.

Surprisingly, it has been found that the stabilized amorphous candesartan cilexetil compositions show improved and enhanced solubility in aqueous systems as compared to the known crystalline forms of candesartan cilexetil. Additionally, the new stabilized amorphous candesartan cilexetil compositions exhibit improved chemical stability including in particular a significant retardation of degradation.

As stabilizing agents, methacrylate based polymers are used. These methacrylate polymers are soluble in acidic aqueous solutions. Preferably, cationic methacrylate polymers are used, selected from the class of aminoalkyl methacrylate polymers and/or copolymers. Most preferably dimethylaminoethyl methacrylate copolymers are used.

Organic solvents in which both the active pharmaceutical ingredient and the stabilizing agents can be dissolved are suitable for the process according to the invention for the preparation of stabilized amorphous candesartan cilexetil compositions. The organic solvents are selected from the group of lower alkanones, alkanols, ethers, esters, aromatic and aliphatic hydrocarbons, aliphatic amides, halogenated hydrocarbons, nitriles, dialkyl sulfoxides as well as mixtures of said solvents. The alkyl moieties of the solvents may contain from one to four carbon atoms. The solvents may be used in their anhydrous form or may contain water. Particularly, the solvents can be selected from the group including acetone, methyl ethyl ketone, methanol, ethanol, isopropanol, n-propanol, 1-butanol, 2-butanol, t-butanol, diethyl ether, dimethyl ether, tetrahydrofuran, dioxane, diisopropyl ether, methyl acetate, ethyl acetate, n-propyl acetate, isopropyl acetate, toluene, xylene, mesitylene, petroleum ether, hexane, cyclohexane, heptane, dichloromethane, chloroform, ethylene dichloride, dimethylformamide, dimethyl acetamide, or acetonitrile. Preferred solvents are ethyl acetate, methyl acetate, acetone, methanol, ethanol and dichloromethane. Most preferred solvent is ethyl acetate.

Candesartan cilexetil and the methacrylate polymer may be dissolved simultaneously in the same organic solvent or sequentially in the same or different organic solvents in either chronological order, and combined. Preferably the methacrylate polymer is dissolved first and the solid candesartan cilexetil is added to the clear solution thus obtained.

According to one preferred embodiment of the present invention the compositions comprise 2 to 75 wt.-% of candesartan cilexetil, based on the total weight of the composition.

The ratio of optionally pharmaceutical active ingredient to methacrylate polymer in the composition according to the invention can be in the range from 5:1 to 1:50, a range from 2:1 to 1:30 being particularly preferred.

Most common pharmaceutical excipients can be used to prepare the pharmaceutical formulations, where tablets are more particularly preferred. As fillers, for example celluloses and cellulose derivatives (for instance microcrystalline cellulose, powdered cellulose, hydroxypropyl cellulose, hydroxypropyl methyl cellulose, methyl cellulose), sugars (for instance lactose, fructose, saccharose, glucose, maltose), sugar alcohols (for instance lactitol, mannitol, sorbitol, xylitol), inorganic fillers (for instance calcium phosphates and calcium sulfates), and starches and starch derivatives (for instance corn starch, potato starch, wheat starch, dextrins, pregelatinized starches) can be used. Beyond that, all other excipients known to those skilled in the art from their basic galenic knowledge, such as lubricants, disintegration aids, wetting agents, agents to improve the flow behaviour, other additives, stabilizers, as well as flavours, pigments, and dyes, can be used to prepare the drug formulations according to the invention (those skilled in the art can obtain the relevant information, for instance, from: Die Tablette, W. A. Ritschel and A. Bauer-Brandl, 2nd ed., ECV-Editio Cantor publishers, 2002).

The weight ratio of the further pharmaceutically acceptable excipient to the methacrylate polymer and candesartan cilexetil in the compositions of the present invention is in a range from 1:10 to 30:1, preferably in the range from 1:5 to 10:1.

Immediate release tablets preferably comprise:
1 to 99 wt.-%, preferably 5 to 99 wt.-% and more preferably 10 to 99 wt.-% of stabilized candesartan cilexetil compositions,
0 to 50 wt.-%, preferably 1 to 25 wt.-% and more preferably 3 to 20 wt.-% disintegration aids,
0 to 50 wt.-%, preferably 1 to 25 wt.-% and more preferably 3 to 20 wt.-% binder,
0 to 95 % wt.-%, preferably 1 to 85 wt.-% and more preferably 20 to 80 wt.-% filler, and
0.1 to 10 wt.-%, preferably 0.1 to 7.5 wt.-% and more preferably 0.1 to 3 wt.-% lubricant.

Additionally, the pharmaceutical formulation may comprise 0.5 to 25wt.-%, preferably 2 to 20 wt.-% and more preferably 1-20 wt.-% of hydrochlorothiazide (HCT).

The process according to the invention yields products of a surprising chemically stability as homogeneous compositions of amorphous candesartan cilexetil.

The stabilized amorphous candesartan cilexetil compositions of the present invention may comprise minor amounts of crystalline candesartan cilexetil. Preferably the stabilized amorphous candesartan cilexetil compositions of the invention comprise less than 5 % of crystalline candesartan cilexetil by weight, even more preferably less than 2 % by weight, based on the total amount of candesartan cilexetil. Most preferably the stabilized amorphous candesartan cilexetil compositions of the present invention comprise no significant amounts of crystalline candesartan cilexetil.

Within this invention, the respective hydrates or solvates of candesartan cilexetil which appear at the end of the synthetic route in solution can also be employed, so that isolation of the pure active ingredient can be avoided.

According to the invention, a process has been found which, starting from a solution of methacrylate based polymer and candesartan cilexetil in an organic solvent, leads to compositions of the active ingredient that can be processed directly for drug formulation.

In one embodiment of the invention, the solution of candesartan cilexetil active ingredient can in principle be prepared by dissolving the active ingredient in a suitable organic solvent; it is more advantageous, however, to use the solution of the active ingredient resulting from the synthetic procedure, without isolation of the candesartan cilexetil.

Candesartan cilexetil can for instance be prepared according to EP 0 459 136 B1, working examples 2 to 8, but omitting the recrystallization steps involving, e.g., dissolving of the product in ethanol and cooling in ice. Instead, the stabilizing material (i.e., the polymethacrylate) can be dissolved in the solution of candesartan cilexetil, before or after the chromatographic purification. Optionally a further pharmaceutically acceptable excipient may additionally be dispersed in the solution. Finally the solvent is removed by drying. The kind of organic solvent used thereby is determined, in any given case, by the final step of synthesis in the process chosen for preparing the active ingredient.

In general, this solution of candesartan cilexetil in an organic solvent is added to a solution of the methacrylate based polymer in an organic solvent, and optionally a pharmaceutically acceptable adjuvant that is insoluble or poorly soluble in the chosen solvents is added. After mixing and dispersion, respectively, the solvent is removed by drying. The drying process can be promoted by heating and/or applying a vacuum, including sublimation drying or spray drying. Advantageously, it is conducted in such a way that appropriate mechanical action (e.g., rotating, tumbling, or stirring motion) yields a uniform distribution. In one embodiment of this invention the organic solution containing the candesartan cilexetil and the methacrylate based polymer is sprayed onto the pharmaceutical acceptable excipients and the solvent is removed as described before. The solvent can be recovered by working in a closed system, and reused for a subsequent process. Compositions prepared by the process described can be employed directly in drug formulations such as tablets, capsules, pellets, or granules, preferably in subsequent processing by a direct compressing process.

Optionally, the compositions or drug formulations thus obtained can be further provided with coatings of pharmaceutical polymethacrylates such as Eudragit^{®} E PO films, methyl celluloses, ethyl celluloses, hydroxypropyl methyl celluloses, cellulose acetate phthalates and/or shellac as required for specific applications, e.g., for controlled release of the active ingredient and/or taste masking. The materials and the process are known to those skilled in the art.

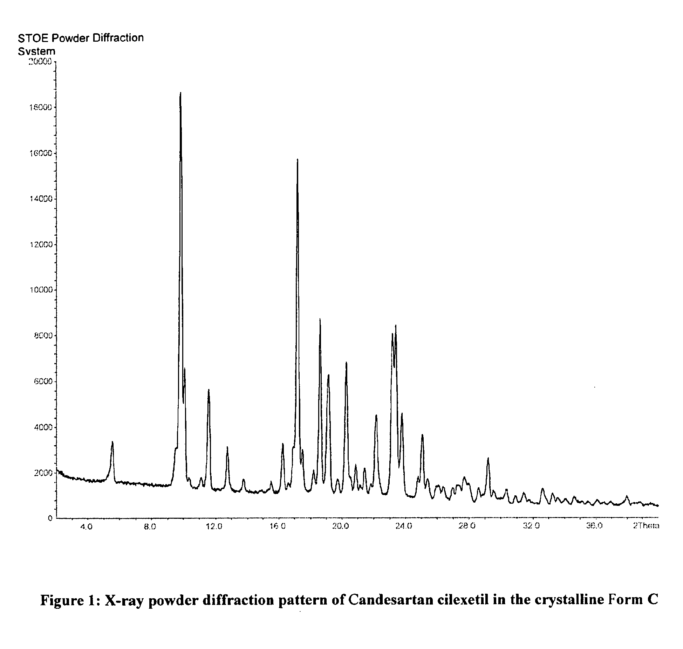

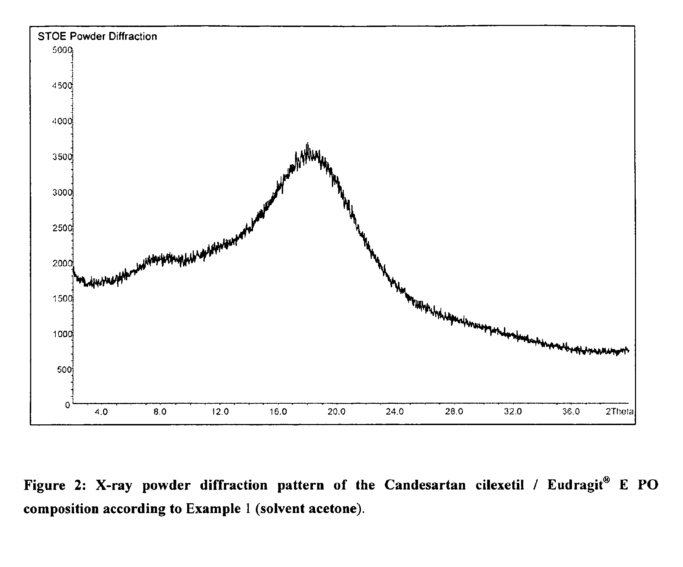

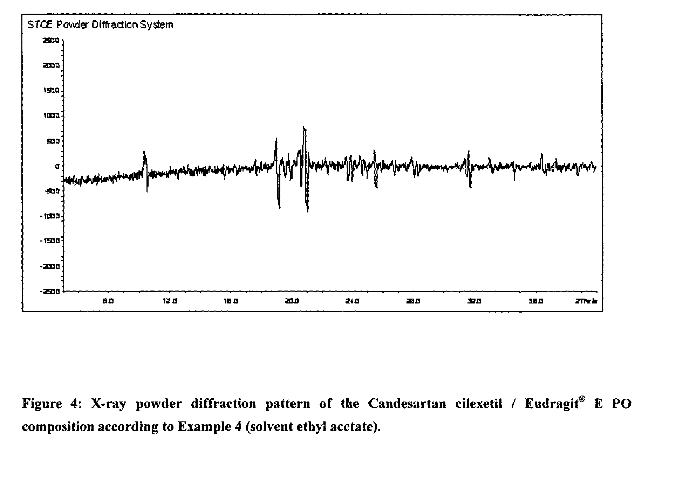

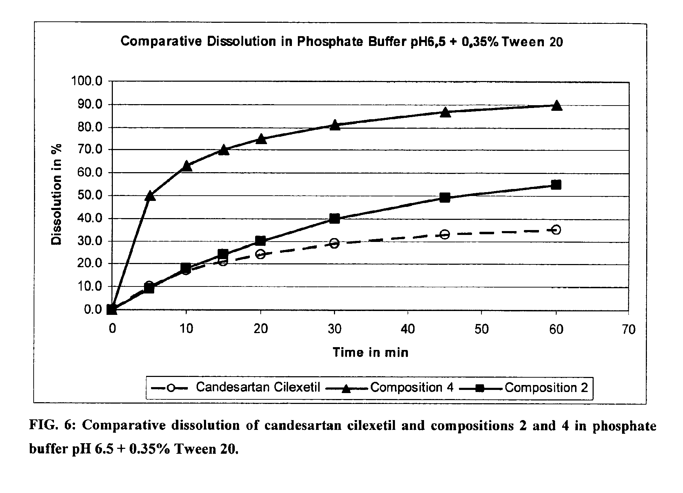

Surprisingly, it has been found that stabilized amorphous candesartan cilexetil compositions prepared by the process according to the invention release the active ingredient without limitations. It appears that the compositions prepared by this process have the active ingredient bound in such a way that the intrinsic crystal structures of the active ingredient can only be poorly developed or preferably not at all. This is demonstrated by X-ray diffraction (see Fig. 2 to 4). For comparison, Fig. 1 shows the powder X-ray diffraction pattern of the crystalline form C of candesartan cilexetil as a typical example of the X-ray diffraction performance of a crystalline polymorph of this compound. The structural characteristics of the amorphous compositions lead to greatly advantageous, enhanced solubility kinetics and thus to a rapid release of the active ingredient (see Fig. 6).

These novel characteristics of the new stabilized amorphous candesartan cilexetil compositions mentioned above are fully retained, when these compositions are processed to drug formulations, e.g., tablets. In particular, direct compressing does not entail any change in morphology and greatly reduces any degradation of candesartan cilexetil during the formulation process.

The invention will now be explained more closely by the following examples and figures, without however limiting the invention thereto.

### EXAMPLES 1 TO 3

### Analytical methods

1. HPLC method for determining the content of active ingredient and survey the contaminants:
   - Instrument: Agilent 1100
   - Buffer:: Dissolve 8.2 g sodium acetate anhydrous in water. Add 10 ml triethylamine and dissolve. Adjust pH to 6.0 with 50% H₃PO₄ and adjust volume to 1000 ml. Filter (0.45 µm) the solution.
   - Mobile phase:: Buffer:acetonitrile (65:35)
   - Column :: Kromasil CN (250mm - 4mm, 5µm)
2. Release of active ingredient (dissolution test): 50rpm, 900ml phosphate buffer pH6.5 including 0.35% Tween 20, 37°C
3. The powder X-ray diffraction patterns were recorded as follows:
   - Instrument:: STADI P transmission diffractometer
   Cu Kα1 radiation (λ = 1.54056 Å), U = 40 kV, I = 30 mA
   Secondary beam monochromator (flat, graphite)
   - Detector:: Scintillation counter
   - Aperture:: 2*8 mm; 0.7 mm; 0.35 mm
   - Linear PSD:: 2 θ = 2° to 35°, 5 s/0.04° in steps
   - Sample:: Powder, reflection mode

### Example 1 : Composition 1

### Candesartan cilexetil : Eudragit^{®} E PO (1:1)

To a solution of 1 g Eudragit^{®} E PO in acetone (20 ml) 1 g of candesartan cilexetil was added. The mixture was stirred until a clear solution was obtained. The solvent was removed under reduced pressure and the composition thoroughly dried.
The X-ray diffraction spectrum is shown in FIG. 2.
The content of O-desethyl candesartan cilexetil impurity is shown in FIG. 5.

### Example 2 : Composition 2

### Candesartan cilexetil : Eudragit^{®} E PO (1:1)

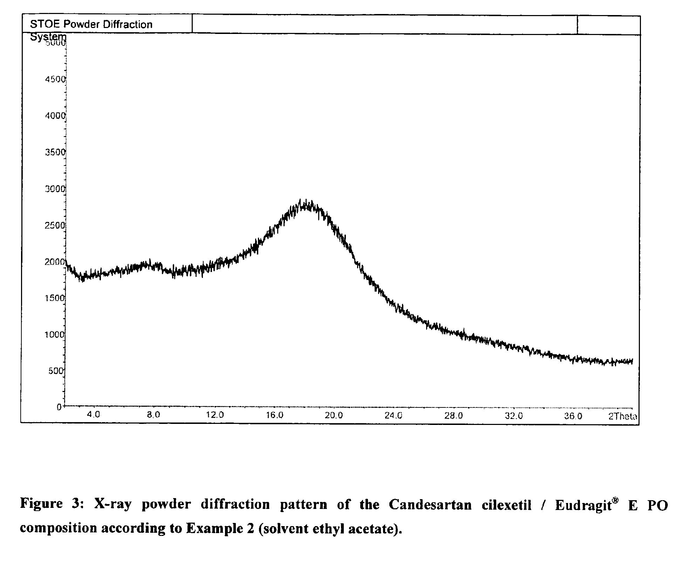

To a solution of 1 g Eudragit^{®} E PO in ethyl acetate (20 ml) I g of candesartan cilexetil was added. The mixture was stirred at 45°C until a clear solution was obtained. The solvent was removed under reduced pressure and the composition thoroughly dried.
The X-ray diffraction spectrum is shown in FIG. 3.
The content of O-desethyl candesartan cilexetil impurity is shown in FIG. 5.
The dissolution profile is shown in FIG. 6.

### Example 3 : Composition 3

### Candesartan cilexetil: Eudragit^{®} E PO: spray-dried lactose (1:1:1)

To a solution of 0.2 g Eudragit^{®} E PO in ethyl acetate (30 ml) 2 g of candesartan cilexetil was added. The mixture was stirred at 45°C until a clear solution was obtained. Then spray dried lactose (4g, Lactopress^{®}) was added. The solvent was removed under reduced pressure and the composition thoroughly dried.
The content of O-desethyl candesartan cilexetil impurity is shown in FIG. 5.

### Example 4 : Composition 4

### Candesartan cilexetil: Eudragit^{®} E PO: spray-dried lactose : microcrystalline cellulose : polyvinylpyrrolidone (1:1:11:0,9:0,4)

To a solution of 2 g Eudragit^{®} E PO in ethyl acetate (50 ml) 2 g of candesartan cilexetil was added. The mixture was stirred at 45°C until a clear solution was obtained. Then spray dried lactose (21g, Lactopress^{®}), microcrystalline cellulose (1.7g, Avicel^{®} PH101), and crosslinked polyvinylpyrrolidone (0.8g, Kollidon^{®} CL) was added. The solvent was removed under reduced pressure and the composition thoroughly dried.
The content of O-desethyl candesartan cilexetil impurity is shown in FIG. 5.
The dissolution profile is shown in FIG. 6.

### FORMULATIONS 1 AND 2

From the compositions, tablets are made by e.g. direct compression using further pharmaceutically acceptable excipients suitable for this task. The relevant information is known to those skilled in the art and can be obtained, for instance, from: Die Tablette, W. A. Ritschel and A. Bauer-Brandes, 2nd ed., ECV-Editio Cantor publishers, 2002.

### Formulation 1

### 8 mg Candesartan cilexetil immediate release tablet

| | |
|---|---|
| Candesartan Cilexetil-Eudragit^{®} E PO composition (1: 1) | 16.0 mg |
| Excipients | 84.0 mg |
| | |
| Tablet weight | 100.0 mg |
| Tablet dimensions | 7 mm, round |
| Disintegration time | 3 min |
| Dissolution (45 min) | ≥ 80 % |

### Formulation 2

### 16 mg Candesartan cilexetil+ 12.5 mg Hydrochlorothiazide immediate release tablet

| | |
|---|---|
| Candesartan cilexetil-Eudragit^{®} E PO composition (1:1) | 32.0 mg |
| Hydrocholorthiazide | 12.5 mg |
| Excipients | 105.5 mg |
| | |
| Tablet weight | 150.0 mg |
| Tablet dimensions | 10 x 4.5 mm, oblong |
| Disintegration time | 3 min |
| Dissolution (45 min) | > 80 % |

The resulting tablets thus obtained can optionallybe further provided with coatings of pharmaceutical polymethacrylates such as Eudragit^{®} E PO films, methyl celluloses, ethyl celluloses, hydroxypropyl methyl celluloses, cellulose acetate phthalates and/or shellac as required for specific applications, e.g., for controlled release of the active ingredient and/or taste masking.

## Claims

1. Stabilized amorphous candesartan cilexetil compositions, comprising amorphous candesartan cilexetil and/or its solvates or hydrates, a stabilizing agent and optionally at least one pharmaceutically suitable excipient, which is insoluble or poorly soluble in organic solvents.

2. Compositions according to claim 1, comprising candesartan cilexetil, or its solvates or hydrates, in a substantially amorphous form.

3. Compositions according to claims 1 and 2, wherein the stabilizing agent is a methacrylate based polymer.

4. Compositions according to claim 3, wherein the methacrylate based polymer is a cationic methacrylate polymer selected from aminoalkyl methacrylate polymers and/or copolymers and preferably is a dimethylaminoethyl methacrylate copolymer.

5. Compositions according to claims 1-4, wherein the weight ratio of active ingredient to stabilizing agent is set in the range from 5:1 to 1:50, more particularly in the range from 2:1 to 1:30.

6. Compositions according to any of the preceding claims, wherein the optional pharmaceutical acceptable excipient is selected from celluloses and cellulose derivatives, lactose, starches and starch derivatives, cyclodextrins, polydextroses or mixtures of said substances and is preferably selected from microcrystalline cellulose, lactose, and mannitol.

7. Compositions according to claim 6, wherein the weight ratio of the mixture of the candesartan cilexetil and the stabilizing agent to the optional pharmaceutical excipient is set in the range from 10:1 1 to 1:30, more particularly set in the range from 5:1 to 1:10.

8. A process for preparing stabilized amorphous candesartan cilexetil compositions according to claim 1 from candesartan cilexetil, in any of its known crystalline forms, in its amorphous form or in solution, and a stabilizing agent soluble in an organic solvent, complemented optionally by at least one pharmaceutically suitable excipient, which is insoluble or poorly soluble in organic solvents.

9. A process according to claim 8 in which candesartan cilexetil and the stabilizing agent are dissolved in an organic solvent.

10. A process according to claim 9 in which candesartan cilexetil and the stabilizing agent are dissolved the same organic solvent or sequentially in the same or different organic solvents in either chronological order.

11. A process according to claim 10, wherein the solvent is selected from acetone, methyl ethyl ketone, methanol, ethanol, isopropanol, n-propanol, 1-butanol, 2-butanol, t-butanol, diethyl ether, dimethyl ether, tetrahydrofuran, dioxane, diisopropyl ether, methyl acetate, ethyl acetate, n-propyl acetate, isopropyl acetate, toluene, xylene, mesitylene, petroleum ether, hexane, cyclohexane, heptane, dichloromethane, chloroform, ethylene dichloride, dimethyl formamide, dimethyl acetamide, or acetonitrile as well as mixtures of the aforementioned solvents.

12. A process according to claim 11, wherein ethyl acetate, methyl acetate, acetone, methanol, ethanol and dichloromethane are used as organic solvents.

13. A process according to claim 11, wherein a solution of candesartan cilexetil is used which is obtained in the final step of the candesartan cilexetil synthesis.

14. A process according to claims 8 to 13, wherein the stabilizing agent is a methacrylate based polymer.

15. A process according to claims 8 to 14, wherein the methacrylate based polymer is a cationic methacrylate polymer selected from aminoalkyl methacrylate polymers and/or copolymers, preferably a dimethylaminoethyl methacrylate copolymer.

16. A process according to claim 14 wherein the weight ratio of candesartan cilexetil to stabilizing agent is set in the range from 5:1 to 1:50, more particularly in the range from 2:1 to 1:30.

17. A process according to any of claims 8-16, wherein the optional pharmaceutical acceptable excipient is selected from celluloses and cellulose derivatives, lactose, starches and starch derivatives, cyclodextrins, polydextroses or mixtures of said substances, preferably from microcrystalline cellulose, lactose, and mannitol.

18. A process according to claim 17, wherein the weight ratio of the mixture of candesartan cilexetil and the stabilizing agent to the optional pharmaceutical excipient is set in the range from 10:1 to 1:30, more particularly in the range from 5:1 to 1:10.

19. Pharmaceutical formulations with at least one active ingredient and optionally further, pharmaceutically acceptable excipients, wherein the formulation contains a candesartan cilexetil composition according to claims 1 to 7 as the at least one active ingredient.

20. Pharmaceutical formulations according to claim 19 in the form of tablets, capsules, pellets, powders and granules obtained, via any of the established processes with common, pharmaceutically acceptable excipients.

21. Pharmaceutical formulations according to claims 19 and 20 in the form of tablets, made by directly compressing the components, which are **characterized by** a rapid release of the active ingredient.

22. Pharmaceutical formulations according to claims 19 to 21, wherein the portion of binder in the total mixture used to prepare the drug is between 1 and 50 wt.%.

23. Pharmaceutical preparations according to claims 19 to 22, wherein the portion of fillers and excipients in the total mixture of the drug preparation is from 1 to 95% by weight, preferably from 1 to 70% by weight.

24. Pharmaceutical preparations according to claims 19 to 23, wherein the formulation additionally comprises 1 to 20 wt.-% of hydrochlorothiazide.

## Amended claims

### Amended claims in accordance with Rule 137(2) EPC.

**1.** Stabilized amorphous candesartan cilexetil compositions, comprising amorphous candesartan cilexetil and/or its solvates or hydrates, an aminoalkyl methacrylate polymer and/or copolymer and optionally at least one pharmaceutically suitable excipient, which is insoluble or poorly soluble in organic solvents.

**2.** Compositions according to claim 1, wherein the aminoalkyl methacrylate copolymer is a dimethylaminoethyl methacrylate copolymer.

**3.** Compositions according to claims 1 or 2, wherein the weight ratio of active ingredient to aminoalkyl methacrylate polymer and/or copolymer is set in the range from 5:1 to 1:50, more particularly in the range from 2:1 to 1:30.

**4.** Compositions according to any of the preceding claims, wherein the optional pharmaceutical acceptable excipient is selected from celluloses and cellulose derivatives, lactose, starches and starch derivatives, cyclodextrins, polydextroses or mixtures of said substances and is preferably selected from microcrystalline cellulose, lactose, and mannitol.

**5.** Compositions according to claim 4, wherein the weight ratio of the mixture of the candesartan cilexetil and the aminoalkyl methacrylate polymer and/or copolymer to the optional pharmaceutical excipient is set in the range from 10:1 to 1:30, more particularly set in the range from 5:1 1 to 1:10.

**6.** A process for preparing stabilized amorphous candesartan cilexetil compositions according to claim 1 from candesartan cilexetil, in any of its known crystalline forms, in its amorphous form or in solution, and an aminoalkyl methacrylate polymer and/or copolymer soluble in an organic solvent, complemented optionally by at least one pharmaceutically suitable excipient, which is insoluble or poorly soluble in organic solvents.

**7.** A process according to claim 6 in which candesartan cilexetil and the aminoalkyl methacrylate polymer and/or copolymer are dissolved in an organic solvent.

**8.** A process according to claim 7 in which candesartan cilexetil and the aminoalkyl methacrylate polymer and/or copolymer are dissolved the same organic solvent or sequentially in the same or different organic solvents in either chronological order.

**9.** A process according to claim 8, wherein the solvent is selected from acetone, methyl ethyl ketone, methanol, ethanol, isopropanol, n-propanol, 1-butanol, 2-butanol, t-butanol, diethyl ether, dimethyl ether, tetrahydrofuran, dioxane, diisopropyl ether, methyl acetate, ethyl acetate, n-propyl acetate, isopropyl acetate, toluene, xylene, mesitylene, petroleum ether, hexane, cyclohexane, heptane, dichloromethane, chloroform, ethylene dichloride, dimethyl formamide, dimethyl acetamide, or acetonitrile as well as mixtures of the aforementioned solvents.

**10.** A process according to claim 9, wherein ethyl acetate, methyl acetate, acetone, methanol, ethanol and dichloromethane are used as organic solvents.

**11.** A process according to claim 10, wherein a solution of candesartan cilexetil is used which is obtained in the final step of the candesartan cilexetil synthesis.

**12.** A process according to claims 6 to 11, wherein the aminoalkyl methacrylate copolymer is a dimethylaminoethyl methacrylate copolymer.

**13.** A process according to claims 6 to 12 wherein the weight ratio of candesartan cilexetil to aminoalkyl methacrylate polymer and/or copolymer is set in the range from 5:1 to 1:50, more particularly in the range from 2:1 to 1:30.

**14.** A process according to any of claims 6-13, wherein the optional pharmaceutical acceptable excipient is selected from celluloses and cellulose derivatives, lactose, starches and starch derivatives, cyclodextrins, polydextroses or mixtures of said substances, preferably from microcrystalline cellulose, lactose, and mannitol.

**15.** A process according to claim 14, wherein the weight ratio of the mixture of candesartan cilexetil and the aminoalkyl methacrylate polymer and/or copolymer to the optional pharmaceutical excipient is set in the range from 10:1 to 1:30, more particularly in the range from 5:1 to 1:10.

**16.** Pharmaceutical formulations with at least one active ingredient and optionally further, pharmaceutically acceptable excipients, wherein the formulation contains a candesartan cilexetil composition according to claims 1 to 5 as the at least one active ingredient.

**17.** Pharmaceutical formulations according to claim 16 in the form of tablets, capsules, pellets, powders and granules obtained, via any of the established processes with common, pharmaceutically acceptable excipients.

**18.** Pharmaceutical formulations according to claims 16 and 17 in the form of tablets, made by directly compressing the components, which are **characterized by** a rapid release of the active ingredient.

**19.** Pharmaceutical formulations according to claims 16 to 18, wherein the portion of binder in the total mixture used to prepare the drug is between 1 and 50 wt.%.

**20.** Pharmaceutical preparations according to claims 16 to 19, wherein the portion of fillers and excipients in the total mixture of the drug preparation is from 1 to 95% by weight, preferably from 1 to 70% by weight.

**21.** Pharmaceutical preparations according to claims 16 to 20, wherein the formulation additionally comprises 1 to 20 wt.-% of hydrochlorothiazide.
